# EUROPEAN PATENT SPECIFICATION

(11) **EP 4 199 829 B1**
(45) Date of publication and mention of the grant of the patent: **28.01.2026**
(21) Application number: 21752761.3
(22) Date of filing: 02.08.2021
(51) Int. Cl.: A61B 17/00

(54) **BIOLOGICS DELIVERY SYSTEM AND MODULAR TIP DESIGN**
BIOLOGISCHES ABGABESYSTEM UND MODULARES SPITZENDESIGN
SYSTÈME D'ADMINISTRATION DE PRODUITS BIOLOGIQUES ET CONCEPTION DE POINTE MODULAIRE

(30) Priority: 20.08.2020 US 202016998769
(43) Date of publication of application: 28.06.2023
(73) Proprietor: Ethicon, Inc., Raritan, NJ 08869 (US)
(72) Inventor: GUO, Jianxin, Raritan, NJ 08869 (US)
(74) Representative: Carpmaels & Ransford LLP
(86) International application number: PCT/IB2021/057065
(87) International publication number: WO 2022/038439

(56) References cited:
- US-A- 5 116 315
- US-A1- 2005 027 240
- US-A1- 2010 310 782
- US-B1- 6 458 095
- US-B1- 6 679 300
- BROWN JIM: "Connection Selection: Identifying Fluid Connector Options", 30 September 2007 (2007-09-30), XP055853506, Retrieved from the Internet <URL:https://cpcworldwide.com/Portals/0/Library/AboutUs/PressRoom/Documents/MDT-ConnectionSelection-Sept07.pdf> [retrieved on 20211021]

## Description

### FIELD

This invention relates to a biologics delivery kit having modular delivery tips, which resists leakage of the biological fluids.

### ENVIRONMENT

In recent years, minimally invasive surgical techniques have emerged as an alternative to conventional surgical techniques to perform a plurality of surgical procedures. Minimally invasive procedures differ from conventional surgical procedures in that a plurality of devices may be introduced into the body through a small incision. As a result, trauma to the body is greatly reduced, thereby decreasing the recovery time of the patient.

One example of a common minimally invasive surgery involves laparoscopic surgical procedures. Laparoscopic procedures may be used to treat hernias, colon dysfunctions, gastroesophageal reflux disease, gallbladder disorders, etc. Typically, the patient undergoing the procedures will return home hours after undergoing surgery.

One challenge presented when performing minimally invasive surgical procedures relates to reducing bleeding at a surgical site when control of bleeding by standard surgical techniques, such as suturing, ligature and cautery, is ineffective or impractical. As opposed to conventional surgical procedures, the surgeon's access to the site of the incision is greatly reduced during minimally invasive procedures and conventional techniques for hemostasis may be difficult to effect.

Recently, the use of tissue sealants and other biological adhesive materials has emerged as an alternate technique for hemostasis. Such tissue sealants may include fibrin, which is comprised of co-reactive thrombin and fibrinogen materials, although other multiple component materials are available. Typically, the individual components of the sealant materials are stored in isolated reservoirs. When mixed, these components may coagulate very quickly, yielding a gel within a short period of time, perhaps 10 or 20 seconds. When applied to the exterior of the body, or when considerable access to the application site is possible, the rapid coagulative properties of the tissue sealant are advantageous. However, such fast-acting properties of conventional tissue sealants have presented potential problems of fouling or clogging during application through laparoscopic devices, which typically results in the destruction of the device.

To protect against cross-contamination, multi-barrel biologics delivery systems have been developed wherein each reactive material is loaded and sealed in a separate vessel, and then brought together into a unified device, such as a mixing/delivery tip, at the time of use without having to transfer the fluid materials into a separate vial or barrel.

Typically, the syringes of such systems are provided with luer-type connectors at the distal ends of the syringes. While luer-type syringe connectors are conventional throughout the health care industry, they do not always provide a leak tight seal against fluid leakage. Additionally, they require a twisting motion to connect and seal with the threads of a conforming device to be attached to the syringe. When side-by-side biologics delivery systems are employed, the twisting motion for locking the luer threads can be awkward.

It would be most convenient to have a syringe/delivery tip design which not only provides improved resistance to liquid leakage, as compared to luer-type connections, but also negates the necessity of the twisting motion necessary to connect the syringes to the delivery tips.

US6458095B1 discusses a dispenser in which a dry powder (one component of a two-part adhesive tissue sealant and/or adhesive) is stored in a container (e.g., a carpule) having a septum at one end, an open end opposite the septum, and a movable plug. The powder is retained at the septum end of the container by the movable plug, which is displaced and pushed back as the solvent used for reconstituting the powder is introduced (e.g., through the septum). The second part of the tissue sealant is contained within a second container, also with a movable plug. After the first part is reconstituted, a manifold is fitted which pierces both septums and allows the contents to be dispensed. A dual syringe body supports the containers, and has pistons that enter the open ends to advance the movable plugs.

US2010/0310782A1 discusses a method and apparatus for applying a sealant including a first chamber that retains a first substance. A second chamber retains a second substance, wherein the second chamber is in selective fluid communication with the first chamber. A third chamber retains a third substance. A first actuator is configured to cause fluid communication of the first and second chambers to allow the first and second substances to comingle and fom1 a first mixture. A second actuator is configured to urge the first mixture and the third substance out of the sealant application apparatus.

US2005/0027240A1 discusses a system for mixing four components that has a first syringe arrangement and a second syringe arrangement. The first and second syringe arrangements each contain at least two chambers for holding one each of the components. It is possible to connect each chamber of one of the syringe arrangements in a detachable fashion to a chamber of the other syringe arrangement. By transferring the components from one of the syringe arrangements into chambers of the other syringe arrangement, the transferred components are mixed with components which are contained in this chamber.

US5116315 discusses a biological syringe system for delivering a first and second fluid in a mixed composition comprising a manifold. The manifold has first and second component channels therethrough and first and second input connections respectively communicating with the first and second component channels. The first and second component channels terminate in exit channels adjacent to each other on an opposing end of the manifold. A discharge assembly is coupled to the opposing end of the manifold and receives fluid from both of the exit channels. The discharge assembly is used for mixing fluid from both of the exit channels and delivering the mixed fluid in a spray. The discharge assembly has a first and second passage therethrough communicating with the exit channels from the manifold for carrying fluid from the first and second component channels in corresponding first and second passages within the discharge assembly while maintaining the first and second fluids separated from each other. The discharge assembly has a mixing space defined therein to receive separate flows from the passages. The discharge assembly further comprises a mixing mechanism disposed within the mixing space to thoroughly mix the first and second fluids for the first time within the mixing space and to immediately thereafter atomize the thoroughly mixed first and second fluids in a spray discharged from the discharge assembly.

Brown, J ("Connection Selection: Identifying Fluid Connector Options", 30th September 2007, XP055853506, URL: https://cpcworldwide.com/Portals/0/Library/AboutUs/PressRoom/Documents/MD T-ConnectionSelection-Sept07.pdf) discusses and reviews available options for fluid connections for medical devices.

### SUMMARY

Provided is a biologics delivery kit, comprising two adjoining syringes, each having a barrel and an exit tube distal to the barrel, and an interchangeable delivery tip, comprising a delivery tip connector portion, and storage cap connectable to the exit tube of each barrel, each having a fixed internal O-ring seal for fluid-tight connection between said delivery tip and storage cap and said exit tube.

In one form, the exit tube does not have a luer connection.

In another form, the biologics delivery system further comprises a locking tab releasably connecting the syringe to the storage cap and delivery tip, such as wherein the locking tab is a flexible tab disposed on the syringes and having a pawl on a distal end thereof to connect to conforming slots in the storage cap and delivery tip connector.

Advantageously, the delivery tip is configured to match the syringes in only one configuration, such as wherein a blocking element located inside the delivery tip connector or storage cap prevents improper connection with the syringe barrels.

In yet another form, the biologics delivery system further comprises a plunger bridge between plungers of said syringes.

In yet another form, the biologics delivery system further comprises a frame to contain said syringes. Advantageously, the frame can comprise a flexible locking tab having a pawl on a distal end thereof and the syringes can be disposed in said frame. In some forms, the frame and syringes can comprise a single part.

In another form, the delivery tip connector can further comprise a manifold.

Additionally presented herein is a method of sealing a biologics delivery system, said system comprising two adjoining syringes, each having a barrel and an exit tube distal to the barrel, said method comprising interchanging a storage cap with a delivery tip having a delivery tip connector portion, each of said storage cap and delivery tip being connectable to the exit tube of each barrel and each having a fixed internal O-ring for fluid-tight connection between said storage cap, said delivery tip and said exit tube.

In one form, the exit tube does not have a luer connection.

In another form, the method further comprises a locking tab releasably connecting the syringe to the storage cap and delivery tip, such as wherein the locking tab is a flexible tab disposed on the syringes and having a pawl on a distal end thereof to connect to conforming slots in the storage cap and delivery tip connector.

Advantageously, the delivery tip is configured to match the syringes in only one configuration, such as wherein a blocking element located inside the delivery tip connector or storage cap prevents improper connection with the syringe barrels.

In yet another form, the method further comprises a plunger bridge between plungers of said syringes.

In yet another form, the method further comprises a frame to contain said syringes. Advantageously, the frame can comprise a flexible locking tab having a pawl on a distal end thereof and the syringes can be disposed in said frame.

### BRIEF DESCRIPTION OF THE DRAWINGS

The present disclosure is susceptible to various modifications and alternative forms, specific exemplary implementations thereof have been shown in the drawings and are herein described in detail. It should be understood, however, that the description herein of specific exemplary implementations is not intended to limit the disclosure to the particular forms disclosed herein.

This disclosure is to cover all modifications and equivalents as defined by the appended claims. It should also be understood that the drawings are not necessarily to scale, emphasis instead being placed upon clearly illustrating principles of exemplary embodiments of the present invention. Moreover, certain dimensions may be exaggerated to help visually convey such principles. Further where considered appropriate, reference numerals may be repeated among the drawings to indicate corresponding or analogous elements. Moreover, two or more blocks or elements depicted as distinct or separate in the drawings may be combined into a single functional block or element. Similarly, a single block or element illustrated in the drawings may be implemented as multiple steps or by multiple elements in cooperation.

The forms disclosed herein are illustrated by way of example, and not by way of limitation, in the figures of the accompanying drawings and in which like reference numerals refer to similar elements and in which:
Figure 1A presents a perspective view of the biologics delivery system of the present disclosure having a storage cap installed on the distal end thereof;
Figure 1B presents a perspective view of the biologics delivery system of the present disclosure having a delivery tip installed on the distal end thereof;
Figure 2A presents a cutaway view of the storage cap according to Fig. 1A;
Figure 2B presents a cutaway view of the delivery tip according to Fig. 1B;
Figure 3 presents the biologics delivery system of Figs. 1A and 1B having both the interchangeable storage cap and delivery tip removed;
Figure 4 presents a perspective view of the delivery tip connector portion of the presently disclosed delivery tips;
Figure 5 presents a cutaway view of the delivery tip connector of Fig. 4; and
Figure 6 presents overall views of several suitable, interchangeable (or modular) delivery tips for use with the biologics delivery system of the present disclosure

### DETAILED DESCRIPTION

Various aspects will now be described with reference to specific forms selected for purposes of illustration. It will be appreciated that the spirit and scope of the apparatus, system and methods disclosed herein are not limited to the selected forms. Moreover, it is to be noted that the figures provided herein are not drawn to any particular proportion or scale, and that many variations can be made to the illustrated forms.

Each of the following terms written in singular grammatical form: "a," "an," and "the," as used herein, may also refer to, and encompass, a plurality of the stated entity or object, unless otherwise specifically defined or stated herein, or, unless the context clearly dictates otherwise. For example, the phrases "a device," "an assembly," "a mechanism," "a component," and "an element," as used herein, may also refer to, and encompass, a plurality of devices, a plurality of assemblies, a plurality of mechanisms, a plurality of components, and a plurality of elements, respectively.

Each of the following terms: "includes," "including," "has," "'having," "comprises," and "comprising," and, their linguistic or grammatical variants, derivatives, and/or conjugates, as used herein, means "including, but not limited to."

Throughout the illustrative description, the examples, and the appended claims, a numerical value of a parameter, feature, object, or dimension, may be stated or described in terms of a numerical range format. It is to be fully understood that the stated numerical range format is provided for illustrating implementation of the forms disclosed herein and is not to be understood or construed as inflexibly limiting the scope of the forms disclosed herein.

Moreover, for stating or describing a numerical range, the phrase "in a range of between about a first numerical value and about a second numerical value," is considered equivalent to, and means the same as, the phrase "in a range of from about a first numerical value to about a second numerical value," and, thus, the two equivalently meaning phrases may be used interchangeably.

It is to be understood that the various forms disclosed herein are not limited in their application to the details of the order or sequence, and number, of steps or procedures, and sub-steps or sub-procedures, of operation or implementation of forms of the method or to the details of type, composition, construction, arrangement, order and number of the system, system sub-units, devices, assemblies, sub-assemblies, mechanisms, structures, components, elements, and configurations, and, peripheral equipment, utilities, accessories, and materials of forms of the system, set forth in the following illustrative description, accompanying drawings, and examples, unless otherwise specifically stated herein. The apparatus, systems and methods disclosed herein can be practiced or implemented according to various other alternative forms and in various other alternative ways.

It is also to be understood that all technical and scientific words, terms, and/or phrases, used herein throughout the present disclosure have either the identical or similar meaning as commonly understood by one of ordinary skill in the art, unless otherwise specifically defined or stated herein. Phraseology, terminology, and, notation, employed herein throughout the present disclosure are for the purpose of description and should not be regarded as limiting.

In the figures, like numerals denote like, or similar, structures and/or features; and each of the illustrated structures and/or features may not be discussed in detail herein with reference to the figures. Similarly, each structure and/or feature may not be explicitly labeled in the figures; and any structure and/or feature that is discussed herein with reference to the figures may be utilized with any other structure and/or feature without departing from the scope of the present disclosure.

Provided herein are new two-component biologics delivery system and delivery tip designs to eliminate the luer connections, which require significant force to seal and are also prone to leak. The modular design for the delivery tips enables all the tip designs with different applications to use the same connection and components to simplify the manufacturing process. The designs also reduce the potential leak interfaces to reduce the likelihood of leaking during application and have features to prevent cross-contamination when users try to detach and reattach tips. The designs also reduce the attachment force between syringes and delivery tips to improve usability.

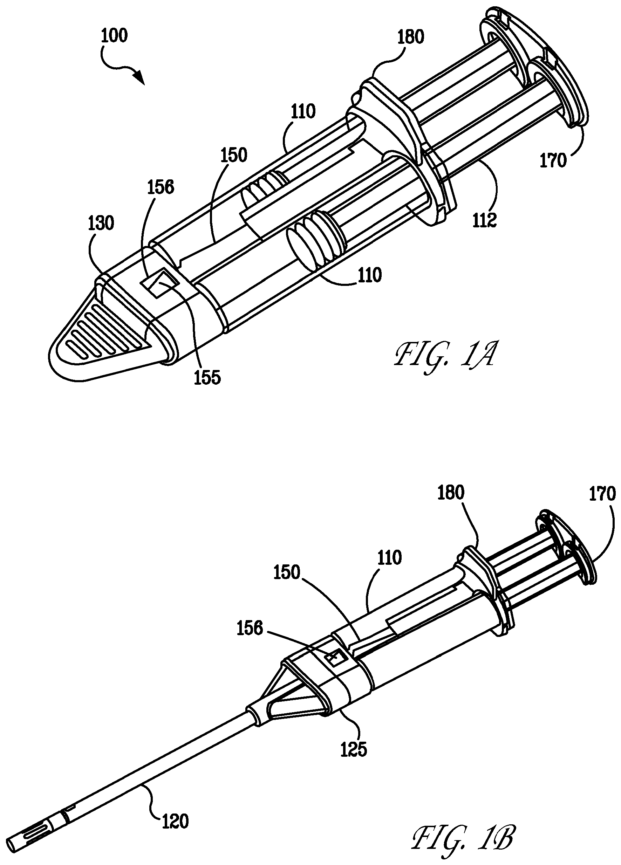

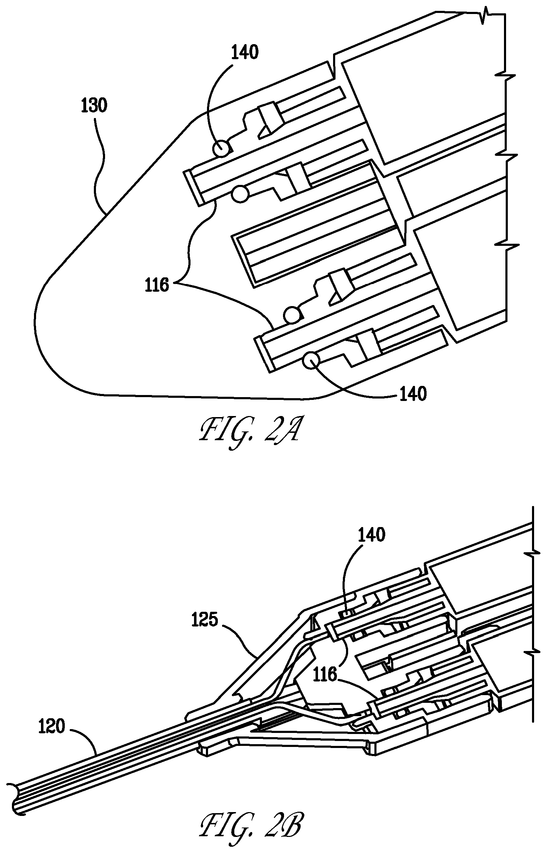

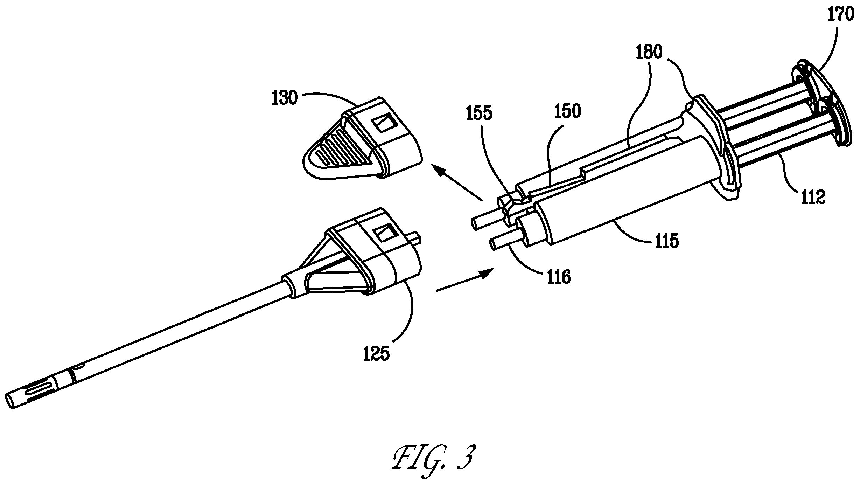

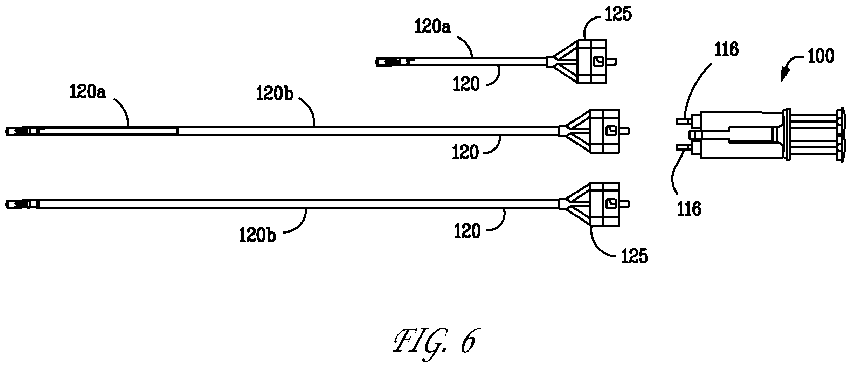

FIGS. 1A through 6 illustrate a biologics delivery system 100, comprising two adjoining syringes 110, each having a barrel 115 and an exit tube 116 distal to the barrel 115, and an interchangeable delivery tip 120, comprising a delivery tip connector portion 125, and storage cap 130 connectable to the exit tube 116 of each barrel, each having a fixed internal O-ring seal 140 for fluid-tight connection between said delivery tip 120, storage cap 130 and said exit tube 116. Conventionally, the syringes 110 incorporate plungers 112 to force the multiple sealing components through the syringes, into the delivery tip 120 and to the surgical situs.

Advantageously, the exit tube 116 portion does not have a luer connection but is instead sealed by application of force against the O-rings 140 disposed within the delivery tip connector 125, and alternatively those disposed within the storage cap 130. The force necessary to seal the O-rings against the exit tube portions of the syringes is considerably less than that necessary to achieve sealing with a luer-type connector.

Once the delivery tip connector 125 is forced against the exit tubes 116, the biologics delivery system 100 has a locking tab 150 releasably connecting the syringe to the storage cap 130 and delivery tip 120, such as wherein the locking tab 150 is a flexible tab disposed on the syringes 110 and having a pawl 155 on a distal end thereof to connect to conforming slots 156 in the storage cap 130 and delivery tip connector 125.

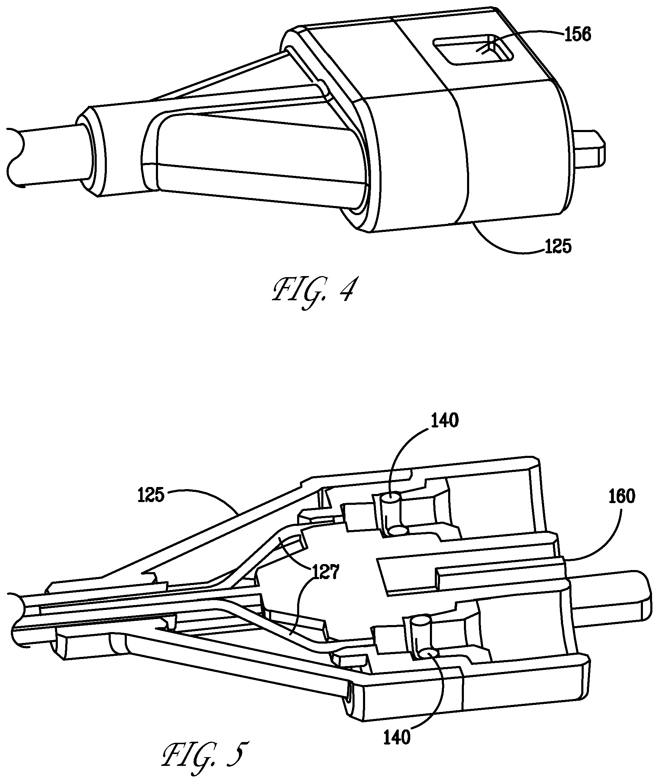

The delivery tip 120 and storage cap 130 are configured to match the biologics delivery system 100 in only one configuration, such as wherein a blocking element 160 located inside the delivery tip connector 125 or storage cap 130 prevents improper connection with the syringe barrels. The blocking element 160 can be configured as an axially off-set, matching key/keyway between the biologics delivery system 100 and the delivery tip connector 125 and storage cap 130, as can be understood from FIG. 5. Other such blocking configurations are known in the art and could be suitably incorporated into the disclosed system.

Advantageously, the biologics delivery system can include a plunger bridge between plungers 112 of said syringes 110, to facilitate consistent delivery from each syringe 110 during use. Additionally, the biologics delivery system can include a frame 180 to contain said syringes 110 in adjoining relationship. Frame 180 can be configured to include the flexible locking tab 150 and pawl 155 on a distal end thereof.

The delivery tip connector can include a manifold 127 therein, to direct the flow of the individual sealing components, such as thrombin and fibrinogen, together and into the delivery tip 120. The presently disclosed connection system is modular, permitting interchangeability between a variety of delivery tips such as open malleable tips 120a, rigid lap tips 120b, and combinations thereof, wherein the tip has both a malleable 120a and rigid 120b portion.

Additionally presented herein is a method of sealing a biologics delivery system 100, said system comprising two adjoining syringes 110, each having a barrel 115 and an exit tube 116 distal to the barrel, said method comprising interchanging a storage cap 130 with a delivery tip 120 having a delivery tip connector portion 125, each of said storage cap 130 and delivery tip 120 being connectable to the exit tube 116 of each barrel 115 and each having a fixed internal O-ring 140 for fluid-tight connection between said storage cap 130, said delivery tip 120 and said exit tube 116. According to the disclosed method, the exit tubes do not have luer connections, which are replaced by the O-ring sealing system.

The method provides for a locking tab 150 releasably connecting the biologics delivery system 100 to the storage cap 130 and delivery tips 120. The locking tab 150 can be a flexible tab disposed on the syringes 110 and having a pawl 155 on a distal end thereof to connect to conforming slots 156 in the storage cap 130 and delivery tip connector 125. In one form, the method provides a frame 180 to contain said syringes 110, into which is provided the flexible locking tab 150 and pawl 155.

The delivery tip 120 is provided with a way to prevent improper connection with the syringe barrels 115, so as to match the biologics delivery system 100 in only one configuration, such as wherein a blocking element 160 located inside the delivery tip connector 125 or storage cap 130 prevents improper connection with the syringe barrels.

Advantageously, the biologics delivery system can include a plunger bridge between plungers 112 of said syringes 110, to facilitate consistent delivery from each syringe 110 during use.

## Claims

1. A biologics delivery kit (100), comprising:
two adjoining syringes (110), each having a barrel (115) and an exit tube (116) distal to the barrel (115);
an interchangeable delivery tip (120), comprising a delivery tip connector portion (125) connectable to the exit tube (116) of each barrel (115), the delivery tip connector portion (125) comprising a first pair of fixed internal O-ring seals (140) for fluid-tight connection between the delivery tip connector portion (125) and said exit tubes (116); and
a storage cap (130) connectable to the exit tube (116) of each barrel (115), the storage cap (130) comprising a second pair of fixed internal O-ring seals (140) for fluid-tight connection between the storage cap (130) and said exit tubes (116).

2. A method of sealing a biologics delivery kit (100), said kit comprising two adjoining syringes (110), each having a barrel (115) and an exit tube (116) distal to the barrel (115), said method comprising interchanging a storage cap (130) with a delivery tip (120) having a delivery tip connector portion (125);
the delivery tip connector portion (125) being connectable to the exit tube (116) of each barrel (115), the delivery tip connector portion (125) comprising a first pair of fixed internal O-ring seals (140) for fluid-tight connection between the delivery tip connector portion (125) and said exit tubes (116); and
the storage cap (130) being connectable to the exit tube (116) of each barrel (115) and the storage cap (130) comprising a second pair of fixed internal O-ring seals (140) for fluid-tight connection between said storage cap (130), and said exit tubes (116).

3. The biologics delivery kit of claim 1 or the method of claim 2, wherein the exit tube does not have a luer connection.

4. The biologics delivery kit of claim 1 or the method of claim 2, wherein the biologics delivery kit of claim 1, or the biologics delivery kit of the method of claim 2 further comprises a locking tab releasably connecting the syringes to the storage cap or delivery tip.

5. The biologics delivery kit of claim 4 or the method of claim 4, wherein the locking tab is a flexible tab disposed on the syringes and having a pawl on a distal end thereof to connect to conforming slots in the storage cap and delivery tip connector.

6. The biologics delivery kit of claim 1 or the method of claim 2, wherein the delivery tip is configured to match the syringes in only one configuration.

7. The biologics delivery kit of claim 6 or the method of claim 6, wherein a blocking element located inside the delivery tip connector or storage cap prevents improper connection with the syringe barrels.

8. The biologics delivery kit of claim 1 or the method of claim 2, wherein each syringe further comprises a plunger and wherein the biologics delivery kit of claim 1, or the biologics delivery kit of the method of claim 2 further comprises a plunger bridge between the plungers of said syringes.

9. The biologics delivery kit of claim 1 or the method of claim 2, wherein the biologics delivery kit of claim 1, or the biologics delivery kit of the method of claim 2 further comprises a frame to contain said syringes.

10. The biologics delivery kit of claim 1 or the method of claim 2, wherein the delivery tip connector portion further comprises a manifold.

11. The biologics delivery kit of claim 9 or the method of claim 9, wherein the frame comprises a flexible locking tab having a pawl on a distal end thereof and the syringes are disposed in said frame.

## Patentansprüche

1. Biologikaabgabekit (100), umfassend:
zwei angrenzende Spritzen (110), die jeweils einen Zylinder (115) und ein Austrittsrohr (116) distal zu dem Zylinder (115) aufweisen;
eine austauschbare Abgabespitze (120), umfassend einen Abgabespitzenverbinderabschnitt (125), der mit dem Austrittsrohr (116) jedes Zylinders (115) verbindbar ist, der Abgabespitzenverbinderabschnitt (125) umfassend ein erstes Paar fester innerer O-Ring-Dichtungen (140) für eine fluiddichte Verbindung zwischen dem Abgabespitzenverbinderabschnitt (125) und den Austrittsrohren (116); und
eine Lagerungskappe (130), die mit dem Austrittsrohr (116) jedes Zylinders (115) verbindbar ist, die Lagerungskappe (130) umfassend ein zweites Paar fester interner O-Ring-Dichtungen (140) für die fluiddichte Verbindung zwischen der Lagerungskappe (130) und den Austrittsrohren (116).

2. Verfahren zum Abdichten eines Biologikaabgabekits (100), das Kit umfassend zwei angrenzende Spritzen (110), die jeweils einen Zylinder (115) und ein Austrittsrohr (116) distal zu dem Zylinder (115) aufweisen, das Verfahren umfassend ein Austauschen einer Lagerungskappe (130) mit einer Abgabespitze (120), die einen Abgabespitzenverbinderabschnitt (125) aufweist;
der Abgabespitzenverbinderabschnitt (125), der mit dem Austrittsrohr (116) jedes Zylinders (115) verbindbar ist, der Abgabespitzenverbinderabschnitt (125) umfassend ein erstes Paar fester innerer O-Ring-Dichtungen (140) für die fluiddichte Verbindung zwischen dem Abgabespitzenverbinderabschnitt (125) und den Austrittsrohren (116); und
die Lagerungskappe (130), die mit dem Austrittsrohr (116) jedes Zylinders (115) verbindbar ist, und die Lagerungskappe (130) umfassend ein zweites Paar fester interner O-Ring-Dichtungen (140) für die fluiddichte Verbindung zwischen der Lagerungskappe (130) und den Austrittsrohren (116).

3. Biologikaabgabekit nach Anspruch 1 oder Verfahren nach Anspruch 2, wobei das Austrittsrohr keine Luer-Verbindung aufweist.

4. Biologikaabgabekit nach Anspruch 1 oder Verfahren nach Anspruch 2, wobei das Biologikaabgabekit nach Anspruch 1 oder das Biologikaabgabekit des Verfahrens nach Anspruch 2 ferner eine Verriegelungslasche umfasst, die die Spritzen mit der Lagerungskappe oder Abgabespitze lösbar verbindet.

5. Biologikaabgabekit nach Anspruch 4 oder Verfahren nach Anspruch 4, wobei die Verriegelungslasche eine flexible Lasche ist, die an den Spritzen angeordnet ist und eine Sperrklinke an einem distalen Ende davon aufweist, um mit konformen Schlitzen in der Lagerungskappe und dem Abgabespitzenverbinder zu verbinden.

6. Biologikaabgabekit nach Anspruch 1 oder Verfahren nach Anspruch 2, wobei die Abgabespitze konfiguriert ist, um mit den Spritzen in nur einer Konfiguration übereinzustimmen.

7. Biologikaabgabekit nach Anspruch 6 oder Verfahren nach Anspruch 6, wobei ein Blockierelement, das sich innerhalb des Abgabespitzenverbinders oder der Lagerungskappe befindet, eine unsachgemäße Verbindung mit den Spritzenkörpern verhindert.

8. Biologikaabgabekit nach Anspruch 1 oder Verfahren nach Anspruch 2, wobei jede Spritze ferner einen Kolben umfasst und wobei das Biologikaabgabekit nach Anspruch 1 oder das Biologikaabgabekit des Verfahrens nach Anspruch 2 ferner eine Kolbenbrücke zwischen den Kolben der Spritzen umfasst.

9. Biologikaabgabekit nach Anspruch 1 oder Verfahren nach Anspruch 2, wobei das Biologikaabgabekit nach Anspruch 1 oder das Biologikaabgabekit des Verfahrens nach Anspruch 2 ferner einen Rahmen umfasst, um die Spritzen zu enthalten.

10. Biologikaabgabekit nach Anspruch 1 oder Verfahren nach Anspruch 2, wobei der Abgabespitzenverbinderabschnitt ferner einen Verteiler umfasst.

11. Biologikaabgabekit nach Anspruch 9 oder Verfahren nach Anspruch 9, wobei der Rahmen eine flexible Verriegelungslasche umfasst, die eine Sperrklinke an einem distalen Ende davon aufweist und die Spritzen in dem Rahmen angeordnet sind.

## Revendications

1. Kit d'administration de produits biologiques (100), comprenant :
deux seringues adjacentes (110), chacune ayant un cylindre (115) et un tube de sortie (116) distal par rapport au cylindre (115) ;
un embout d'administration interchangeable (120), comprenant une partie raccord d'embout d'administration (125) pouvant être raccordée au tube de sortie (116) de chaque cylindre (115), la partie raccord d'embout d'administration (125) comprenant une première paire de joints toriques internes fixes (140) pour un raccordement étanche aux fluides entre la partie raccord d'embout d'administration (125) et lesdits tubes de sortie (116) ; et
un bouchon de stockage (130) pouvant être raccordé au tube de sortie (116) de chaque cylindre (115), le bouchon de stockage (130) comprenant une seconde paire de joints toriques internes fixes (140) pour un raccordement étanche aux fluides entre le bouchon de stockage (130) et lesdits tubes de sortie (116).

2. Procédé de scellement d'un kit d'administration de produits biologiques (100), ledit kit comprenant deux seringues adjacentes (110), chacune ayant un cylindre (115) et un tube de sortie (116) distal par rapport au cylindre (115), ledit procédé comprenant l'échange d'un bouchon de stockage (130) avec un embout d'administration (120) ayant une partie raccord d'embout d'administration (125) ;
la partie raccord d'embout d'administration (125) pouvant être raccordée au tube de sortie (116) de chaque cylindre (115), la partie raccord d'embout d'administration (125) comprenant une première paire de joints toriques internes fixes (140) pour un raccordement étanche aux fluides entre la partie raccord d'embout d'administration (125) et lesdits tubes de sortie (116) ; et
le bouchon de stockage (130) peut être raccordé au tube de sortie (116) de chaque cylindre (115) et le bouchon de stockage (130) comprend une seconde paire de joints toriques internes fixes (140) pour un raccordement étanche aux fluides entre ledit bouchon de stockage (130) et lesdits tubes de sortie (116).

3. Kit d'administration de produits biologiques selon la revendication 1 ou procédé selon la revendication 2, dans lequel le tube de sortie n'a pas de raccord Luer.

4. Kit d'administration de produits biologiques selon la revendication 1 ou procédé selon la revendication 2, dans lequel le kit d'administration de produits biologiques selon la revendication 1 ou le kit d'administration de produits biologiques du procédé selon la revendication 2 comprend en outre une languette de verrouillage reliant de manière libérable les seringues au bouchon de stockage ou à l'embout d'administration.

5. Kit d'administration de produits biologiques selon la revendication 4 ou procédé selon la revendication 4, dans lequel la languette de verrouillage est une languette flexible disposée sur les seringues et ayant un cliquet sur une extrémité distale de celle-ci pour se relier à des fentes conformes dans le bouchon de stockage et le raccord d'embout d'administration.

6. Kit d'administration de produits biologiques selon la revendication 1 ou procédé selon la revendication 2, dans lequel l'embout d'administration est conçu pour s'assembler avec les seringues dans une seule configuration.

7. Kit d'administration de produits biologiques selon la revendication 6 ou procédé selon la revendication 6, dans lequel un élément de blocage situé à l'intérieur du raccord d'embout d'administration ou du bouchon de stockage empêche une liaison incorrecte avec les seringues.

8. Kit d'administration de produits biologiques selon la revendication 1 ou procédé selon la revendication 2, dans lequel chaque seringue comprend en outre un piston et dans lequel le kit d'administration de produits biologiques selon la revendication 1 ou le kit d'administration de produits biologiques du procédé selon la revendication 2 comprend en outre un pont de pistons entre les pistons desdites seringues.

9. Kit d'administration de produits biologiques selon la revendication 1 ou procédé selon la revendication 2, dans lequel le kit d'administration de produits biologiques selon la revendication 1 ou le kit d'administration de produits biologiques du procédé selon la revendication 2 comprend en outre un cadre pour contenir lesdites seringues.

10. Kit d'administration de produits biologiques selon la revendication 1 ou procédé selon la revendication 2, dans lequel la partie raccord d'embout d'administration comprend en outre un collecteur.

11. Kit d'administration de produits biologiques selon la revendication 9 ou procédé selon la revendication 9, dans lequel le cadre comprend une languette de verrouillage flexible ayant un cliquet sur une extrémité distale de celle-ci et les seringues sont disposées dans ledit cadre.
